# EUROPEAN PATENT APPLICATION

(11) **EP 1 577 011 A2**
(43) Date of publication of application: **21.09.2005**
(21) Application number: 05250907.2
(22) Date of filing: 17.02.2005
(51) Int. Cl.: B01L 3/14, C12M 1/33

(54) **Disruption of cells and tissues**

(30) Priority: 18.02.2004 US 781036
(71) Applicant: Veridex, LLC, Warren, NJ 07059 (US)
(72) Inventor: Belly, Robert, Webster, NY 14580 (US); Hays, Dustin, Bethesda, MD 20817-3753 (US)
(74) Representative: Mercer, Christopher Paul

(57) **Abstract**

A method for disrupting cells and/or tissues employs a disrupter with a disruption element having an outer dimension that is slightly smaller than the inside dimension of a container in which a sample is place to be disrupted. Devices for disrupting cells and/or tissues are also presented. The device and methods are used in processes such as the extraction of nucleic acids.

SEQUENCE LISTING

<110>  Veridex, LLC
       Veridex, LLC
       Belly, Robert
       Hays, Dustin

<120>  Disruption of Cells and Tissues

<130>  VDX-5001 USNP

<160>  3

<170>  PatentIn version 3.1

<210>  1
<211>  20
<212>  DNA
<213>  Artificial

<220>
<223>  Artificial Sequence is  a Primer

<400>  1
ccacacggtg cccatctacg                                                  20


<210>  2
<211>  26
<212>  DNA
<213>  Artificial

<220>
<223>  Artificial Sequence is a Primer

<400>  2
aggatcttca tgaggtagtc ggtcag                                           26


<210>  3
<211>  30
<212>  DNA
<213>  Artificial

<220>
<223>  Artificial Sequence is a Probe

<400>  3
acgccctgcc ccacgccatc ctgcgtctgg                                       30

## Description

### Background

RNA expression can be used as the basis of *in vitro* diagnostic tests useful in screening, diagnosis, prognosis, therapeutic monitoring, and/or follow-up of relapse of disease. Intact non-degraded RNA extracted from cells or tissues is important for the valid measurement of RNA expression. RNA expression is particularly useful in microarray and PCR assays involving numerous genes.

In most RNA extraction methods, RNA is released from cells by mechanical means. Often, this is done in the presence of RNA stabilizing agents such as guanidium salt. This minimizes the degradation of RNA by RNAases released from the cell or tissue during the process. Devices for the mechanical disruption of biological samples are proposed in the following exemplary US patents: 4,307,846 to Spelsberg; 5,197,483 to Rogalsy; 5,840,878 to Collis; 5,829,696 to DeStefano; 6,235,501 to Gautsch; and 5,567,050 to Zoblinsky. In such devices, an element is used to grind, pulverize, or otherwise contact the sample containing the cellular component that is to be extracted. Throughout this specification, this element is referred to as a disruption element. Most often the devices use a disruption element that is a bead or ball such as a glass bead.

There is a continuing need to improve cell and tissue disruption methods so that RNA extraction can be made faster, more efficient, more productive, and amenable to automation. This is particularly desirable in the case of intra-operative assays.

### Summary of the Invention

The invention is a method of disrupting cells and tissues. Preferably, the method involves the use of a mechanical device and is conducted in 45 seconds or less.

Another aspect of the invention is a method of extracting RNA from a cell or tissue sample. Preferably, the sample is a lymph node tissue. In the method, cells and/or tissues are mechanically disrupted, preferably in 45 seconds or less. Cellular debris and other non-RNA materials such as air bubbles are then removed from the sample exposed to the disruption. The RNA is then extracted from the sample.

In yet another aspect of the invention, a device for disrupting cells and tissues includes a containment portion in which a disruption element is placed. The space of the outer surface of the disrupter (circumference in the case of a bead) is just slightly smaller than the inner dimension of the containment portion (diameter in the case of a tube).

### Detailed Description

In the most preferred embodiment of the invention, a mechanical cell and tissue disrupter such as the "FASTPREP" instrument available from Qbiogene, Inc. of Carlsbad, CA is used. Such disrupters are described in US Patent 5,567,050. Preferably, the method of the invention employs 2.0 ml screw-capped cryovial tubes containing an "O-ring", or their equivalent. Between about 30 mg and 400 mg of tissue are placed in the tubes along with a disrupter element. Most preferably, the disrupter elements are steel beads having a diameter of 6.35mm such as those available from BioSpec Products of Bartelesville, OK. To the tube, is added a volume of homogenization buffer (e.g., RLT buffer from Qiagen Corp.) that is about half of the tube or more. Preferably, about 1ml of such buffer is added when using the 2.0 ml tube. The homogenzation buffer contains an RLT stabilizer, preferably a guanidium salt. The disrupter is then engaged for 45 seconds or less and the sample is centrifuged, preferably in a microfuge, to remove the cellular debris and air bubbles from the samples. The supernatant is then decanted. Preferably, the supernatant is transferred into a spin column and the column is treated with different wash buffers to purify RNA. Solvent-based RNA purification methods can be used in place of the spin column if desired too.

The device of the instant invention is a disrupter such as one described above but the dimensions and/or composition of the disruption element are provided that rapidly and efficiently disrupt the tissue in a way not previously possible. As described above, the disruption element is the portion of the device that makes contact with the tissue. Preferably, this is a bead. The disruption element of the invention has an outer dimension that is just slightly smaller than the inner dimension of the container in which the sample is processed. In the preferred embodiment, the container is a tube such as the one described above with an inner diameter of 8.28 mm and the disruption element is a 6.35mm steel bead. Other containers and disruption elements can also be used provided that their inner and outer dimensions are of similar proportions. The density of the bead also contributes to the improved performance of the inventive device. Preferably, the bead is made of stainless steel with a density of 8.03 g/cm³ or greater although beads having a density above 7.0 can be used.

### Examples

### Example 1. Evaluation of different bead sizes and composition

Different bead sizes and materials were evaluated for their ability to disrupt frozen pig lymph node tissue. Two ml screw-cap tubes (BioSpec Products, Bartlesville, OK) with an 8.28mm inner diameter were used. Each tube was half-filled with beads except for tubes containing a 6.35mm (0.25 inch) stainless steel beads, in which case a single bead was added per tube. Between 200 and 250 mg of frozen, pulverized pig lymph node tissue was added per tube. The tubes were filled with Buffer RLT (Qiagen, Inc, Valencia CA) and homogenized for 1, 3, or 5 min by means of a Mini-Bead Beater 8 instrument obtained from BioSpect Products).

The following bead sizes and compositions were purchased from Biospec Products : 0.1 mm diameter zirconia/silica (cat 11107901 z), 0.1mm diameter glass (cat 11079101), 0.5mm diameter glass (cat 1107905), 0.5mm diameter zirconia/silica (cat 11079105z), 1.0mm diameter glass (cat 11079110), 2.5mm diameter zirconia/silica (cat 11079125z), 6.35 mm diameter stainless steel (cat 11079635ss), and 6.35 mm diameter glass beads (110796325).

Results of these studies are shown in Table 1 below and indicated best tissue disruption with multiple 6.35 mm diameter glass beads or a single 6.35mm stainless steel bead, as determined visually with the following scale: (1) poorly homogenized tissue, (2) partially homogenized tissue, and (3) completely homogenized tissue.

**Table 1.**

| Evaluation of Different Bead Size and Composition on Node Tissue homogenization* | | | | | | |
|---|---|---|---|---|---|---|
| | **Node tissue** | **Bead** | **Bead** | **Tissue Disruption** | | |
| **Tube no.** | **wt (mg)** | **size (mm)** | **Composition** | **1min** | **3min** | **5min** |
| 1 | 207 | 0.1 | zirconia/silica | 1 | 1 | 1 |
| 2 | 221 | 0.1 | glass | 1 | 1 | 2 |
| 3 | 203 | 0.5 | glass | 1 | 1 | 1 |
| 4 | 204 | 0.5 | zirconia/silica | 1 | 1 | 1 |
| 5 | 225 | 1 | glass | 1 | 1 | 1 |
| 6 | 249 | 2.5 | zirconia/silica | 1 | 2 | 3 |
| 7 | 200 | 6.35 | stainless | ND | 2 | 3 |
| 8 | 230 | 6.35 | glass | 2 | 2 | 3 |

| | | | | | | |
|---|---|---|---|---|---|---|
| *Tissue disruption was determined visually as described in the text above. | | | | | | |

### Example 2. Effect of Bead Density

Example 1 illustrates that complete tissue homogenization is best achieved using a disruption element (bead) of 6.35mm in diameter. Bead density also affects the quality of homogenization.

A comparison was made between beads possessing equal diameter (6.35mm) but different densities. Approximately 225mg of pig lymph node was disrupted in 2ml screw-cap vials containing 1ml of a guanidinium-based lysis buffer (Qiagen Buffer RLT), plus one 6.35mm bead of ceramic, glass, or stainless steel. Tissue samples containing beads were processed for 1 minute, 3 minutes, and 5 minutes in the Biospec Bead Beater 8. Samples were briefly centrifuged and were assessed visually for quality of homogenization as in Example 1.

**Table 2.**

| Performance of 6.35mm Beads of Various Densities on the Homenization of Pig Lymph Node | | | | | | | |
|---|---|---|---|---|---|---|---|
| | | | | | **Tissue Disruption** | | |
| **Bead size/type** | **Bead Weight (g)** | **Bead Density mg/mm3** | **Manufacturer / catalog #** | **Avg. Tissue Weight** | **1 min** | **3 min** | **5 min** |
| 6.35mm glass | 276 | 2.74 | Biospec/ 11079635 | 248 | 1.00 | 1.67 | 2.00 |
| 6.35mm ceramic | 679 | 6.75 | Q Biogene cat. # 6910-052 | 243 | 2.33 | 3.00 | 3.00 |
| 6.35 mm stainless steel | 1008 | 10.02 | Biospec/ 11079635ss | 226 | 2.67 | 3.00 | 3.00 |

Table 2 summarizes the average of 3 replicates scored visually using the following scale: (1) poorly homogenized tissue, (2) partially homogenized tissue, and (3) completely homogenized tissue. This experiment shows that tissue disruption improves as the density of bead material increases, with the best performance achieved with stainless steel beads.

### Example 4. RNA Yield and Purity with the BeadBeater Instrument and Glass or Stainless Beads

RNA yield and purity were determined with either glass or stainless steel beads of several diameters of 6.35mm or greater. The Mini-Bead Beater 8 was used at high speed for 1 min. Conditions were as described previously in example 1. Stainless steel and glass beads 6.35mm were obtained from Biospec Products. All other beads were purchased from Glen Mills Inc, Clifton, NJ.

After tissue disruption, the resultant homogenate was centrifuged 15 sec by means of an Eppendorf model 5415 (Brinkman Instruments, Westbury NY), and RNA was purified by means a QIAGEN RNeasy Mini kit (Qiagen Inc, Valencia CA) A portion of the lysate was diluted to a concentration of 30mg tissue/ml of Buffer RLT. Four hundred microliters of lysate was added to a microfuge tube containing 400ul of 70% ethanol, and the sample was mixed by pipetting. Sample (700ul) was applied to an RNeasy mini column placed on a vacuum manifold. Vacuum was applied and maintained during the remainder of the filtration steps. The column was washed with 700ul of Buffer RWI, followed by 700ul of Buffer RPE. The spin column was then placed in a 1.5ml collection tube, and centrifuged at 14,000 RPM for 30 seconds to dry the column. The column was transferred to a new 1.5ml collection tube. Fifty microliters of RNAase-free water was applied directly to the membrane, and the tube containing the column was centrifuged for 30 seconds at 14,000 RPM to elute the RNA. The RNA samples were placed on ice, and stored at -80C prior to assaying.

RNA yield was measured spectrophotometrically based on the 260/280nm ratio of the sample as determined on a Gene Spec III instrument (MiraiBio, Alameda CA). An Agilent 2100 Bioanalyzer and Agilent RNA 6000 Nano Assay Reagents and Supplies (Agilent Technologies, Foster City, CA.) were also used to determine RNA yield and quality.

Results are summarized in Table 3, and demonstrate that a stainless steel bead 6.35mm in diameter provides the highest RNA yield, and best tissue homogenization as determined by visual inspection of the tubes. Furthermore, a stainless steel bead, as compared to a glass bead of identical diameter, shows significant improvement in yield. Agilent Bioanalyzer electropherograms confirm that high quality RNA was obtained with the rapid homogenization method. Clearly defined 18s and 28s ribosomal peaks were shown in the samples disrupted by the rapid bead based method, and all samples had an 18s to 28s ratio greater than 1.5.

**Table 3.**

| **RNA Yield with Different Size and Composition *** | | | | | |
|---|---|---|---|---|---|
| **Tube no.** | **Node Tissue** | **Bead** | **Bead** | **Homogenization** | **RNA yield** |
| | **wt (mg)** | **diameter (mm)** | **Composition** | **1 min** | **ug RNA/mg tissue** |
| 1 | 480 | 6.35 | Stainless | 4 | 0.069 |
| 2 | 400 | 6.35 | Stainless | 4 | 0.067 |
| 3 | 400 | 6.35 | Stainless | 3 | 0.073 |
| 4 | 392 | 6.35 | Stainless | 3 | 0.053 |
| 5 | 454 | 6.35 | Stainless | 4 | 0.085 |
| 6 | 456 | 6.35 | Stainless | 4 | 0.043 |
| 7 | 422 | 6.35 | Glass | 2 | 0.03 |
| 8 | 445 | 6.35 | Glass | 2 | 0.026 |
| 9 | 481 | 6.35 | Glass | 2 | 0.022 |
| 10 | 473 | 6.35 | Glass | 2 | 0.032 |
| 11 | 378 | 6.35 | Glass | 2 | 0.041 |
| 12 | 401 | 6.35 | Glass | 2 | 0.035 |
| 13 | 463 | 7 | Stainless | 4 | 0.018 |
| 14 | 503 | 8 | Stainless | 2 | 0.023 |
| 15 | 389 | 7.93 | Stainless | 2 | 0.027 |
| 16 | 537 | 7.14 | Stainless | 2 | 0.067 |

| | | | | | |
|---|---|---|---|---|---|
| *Tissue disruption was measured visually based on the following scale: 1 homogenization, 2-poor homogenization, 3-partial homogenization (few pieces of tis remain), 4-visible cell debris, and 5-complete homogenization. | | | | | |

### Example 3. Tissue disruption in the FastPrep Instrument

The following experiment was performed to determine the precision of RNA yield for pig node samples weighing between 50 and 500mg when disrupted in the FastPrep Homogenizer (Qbiogene, Inc, Carlsbad CA). These experiments were performed with 2ml Sarstedt screw-cap vials containing a single 6.35mm stainless steel bead.

In these experiments, different weights of frozen and pulverized pig lymph node tissue shown in Table 4 were added into 2ml Sarstedt screw-cap vials containing one 6.35mm stainless steel bead per tube. One ml of Buffer RLT was added and the tubes were processed for 45 sec in a FastPrep instrument with a speed of 6.5meters/sec. (To perform this experiment, the FastPrep instrument was modified by removing washers from the spokeplate so that this larger tube size could be accommodated). After disruption, tubes were then centrifuged at 14,000 x g for 15 sec in an Eppendorf model 5415C (Brinkman Instruments, Westbury, NY) to pellet cellular debris and remove air bubbles from the sample. A portion of each lysate was diluted to a concentration of 30mg of tissue/ml of Buffer RLT, and mixed well by pipetting. The tissue was processed to extract RNA, and RNA was quantified in Example 3.

**Table 4.**

| **Effect of Node Weight on RNA yield and Precision** | | | | | | | |
|---|---|---|---|---|---|---|---|
| | | | | | | | |
| **Experimental condition** | **Tube #** | **Tissue Weight (mg)** | **260/280 ratio** | **Total RNA (ug)** | **Total RNA mean recovery(ug)** | **SD** | **%CV** |
| 50-100mg tissue | 1 | 53 | 2.1 | 13.1 | 24.3 | 6.8 | 0.28 |
| | 2 | 54 | 2.1 | 20.6 | | | |
| | 3 | 79 | 2.0 | 27.2 | | | |
| | 4 | 80 | 2.1 | 24.5 | | | |
| | 5 | 50 | 2.1 | 27.8 | | | |
| | 6 | 67 | 2.0 | 32.8 | | | |
| 300-400mg tissue | 19 | 357 | 2.0 | 23.7 | 22.2 | 1.3 | 0.06 |
| | 20 | 359 | 2.0 | 22.1 | | | |
| | 21 | 383 | 2.0 | 20.2 | | | |
| | 22 | 357 | 2.1 | 22.1 | | | |
| | 23 | 367 | 2.0 | 23.7 | | | |
| | 24 | 325 | 2.1 | 21.6 | | | |
| 400-500mg tissue | 25 | 456 | 1.9 | 19.3 | 17.3 | 3.3 | 0.19 |
| | 26 | 470 | 2.0 | 18.4 | | | |
| | 27 | 414 | 1.8 | 19.1 | | | |
| | 28 | 442 | 2.0 | 18.9 | | | |
| | 29 | 468 | 2.0 | 17.5 | | | |
| | 30 | 444 | 2.1 | 10.8 | | | |

Results of these studies demonstrate total RNA recoveries of over 20ug at samples less than 400mg of tissue. Slightly less total RNA recovery was observed in pig node samples between 400 and 500mg, suggesting slightly less efficient tissue disruption under these experimental conditions.

Tubes containing tissue samples of lower weight had the highest imprecision. However, in subsequent experiments, we have shown that higher standard deviations at lower node tissue weights are probably due to tissue heterogeneity and sampling since the precision of tissue recovery improves significantly when a larger tissue is pulverized and then a smaller tissue weight is used for homogenization.

### Example 5. Real-time PCR assay of Porcine B-actin gene expression

The following experiment was performed to demonstrate that RNA recovered after rapid homogenization in the Fast Prep method is amplifiable in real-time PCR assays. Purified RNA from example 4 was used. For comparison, a control was included in which pig node tissue was disrupted by mechanical disruption by means of an Omni Tissue Homogenizer and disposable PCR probes (Omni International, Warrenton, VA). The Omni tissue homogenizer does not use beads to enhance tissue disruption. For treatment in the Omni Tissue homogenizer, 100-400 mg of pig node tissue was mixed with 6 ml of Buffer RLT and homogenized with a hand homogenizer (model PCR258) for 1 min. Samples were mixed with an equal volume of 70% ethanol, and 700ul was applied to a Qiagen spin column and RNA was purifed as described in Example 3.

RNA was transcribed to make cDNA copies as follows: One µg of RNA was added to a master mix made by mixing 1.25µl of a 75µM stock solution of anchored Oligo dT23, 0.31µl of 10mM of each dNTP, and RNAase -treated water (Sigma Chemical Co, St. Louis, Mo.) to a volume of 13µL ,and the solution was heated to 70C for 5 min, and then placed on ice. Then a 9.5µL mix comprising 5µL of 5X Superscript First-Strand Buffer, 2.5µL of 0.1M Dithiothreitol, 0.75µL of a solution of 40U/µL Rnasin (Promega Corp, Madison WI) and 1.25 µL of 200U/µL Superscript II reverse transcriptase was added, and the tube was incubated at 42C for 50min. FiveµL of 0.5M NaOH was added, the tube was incubated at 70C for 5min, and then Tris-HCl buffer, (2.5µl of 1M, pH 7.0), was added, followed by 67.4µl of RNAase free water. Assuming a complete conversion of RNA to cDNA, and a conversion factor of 1ug equal to 50,000 cell equivalents (CE), a concentration of 500CE/µL in 25mM Tris buffer was used for all subsequent real-time PCR assays.

Porcine B-actin primers (SEQ ID NO. 1 and SEQ ID NO. 2) were synthesized by Invitrogen Corp.(Carlsbad, CA) and the B-actin Taqman probe (SEQ ID NO. 3) from. SYNTHEGEN, LLC (Houston, TX). For all Taqman probes, carboxyfluorescein (FAM) and carboxytetramethylrhodamine TAMRA) were used as the dye and quencher pair.

SEQ ID NO. 1 CCACACGGTG CCCATCTACG

SEQ ID NO. 2 AGGATCTTCA TGAGGTAGTC GGTCAG

SEQ ID NO. 3 6-FAM –ACGCCCTGCC CCACGCCATC CTGCGTCTGG- TAMRA

For the Taqman assays, a master mix was prepared by adding 25µl of 2X Taqman Universial PCR mix (Applied Biosystems, Inc), 1.25µl of a 10µ M stock probe solution, and 5µl of a 0.5µ M solution of each primer. cDNA (2µl) was added to each optical reaction microtiter plate well. After capping with optical caps, the plates were processed in an ABI Prism 7900 HT Sequence Detection System (Applied Biosystems, Inc., Foster City, CA). and the assay was performed according to the protocol recommended by the manufacturer. An average of three determinations by Taqman assays are shown in Table 5.

Results of these are summarized in Table 5, and demonstrate that comparable yields of RNA can be obtained with the method of this invention and the Omni Homogenizer. The method of the present invention is advantageous in that multiple samples can be processed simultaneously. Furthermore, since sealed tubes are used, sample to sample contamination is minimized.

**Table 5.**

| **Taqman Assay of RNA Homogenized by Omni Homogenizer and by Fast Prep Instrument** | | | | | |
|---|---|---|---|---|---|
| **Tissue Homogenization** | **Node Size (mg)** | **Avg Ct** | **SD** | **%CV** | **Avg** |
| Omni Homogenizer | 124 | 20.11 | 0.47 | 0.02 | |
| | 129 | 19.95 | 0.36 | 0.02 | |
| | 113 | 19.8 | 0.09 | 0 | |
| | 94 | 20.02 | 0.07 | 0 | 19.97 |
| | 394 | 18.79 | 1.19 | 0.06 | |
| | 388 | 19.57 | 0.31 | 0.02 | |
| | 392 | 19.01 | 0.76 | 0.04 | |
| | 412 | 19.96 | 0.24 | 0.01 | 19.33 |
| Fastprep Instrument | 80 | 20.33 | 0.65 | 0.03 | |
| | 145 | 19.48 | 0.5 | 0.03 | |
| | 142 | 20.39 | 0.17 | 0.01 | |
| | 123 | 20.88 | 0.3 | 0.01 | 20.27 |
| | 456 | 19.89 | 0.29 | 0.01 | |
| | 470 | 20.03 | 0.27 | 0.01 | |
| | 414 | 20.03 | 0.34 | 0.02 | |
| | 442 | 20.85 | 0.42 | 0.02 | 20.2 |

## Claims

1. A device comprising a cell or tissue disrupter having a disruption element for use in conjunction with a sample container wherein the disruption element has an outer dimension slightly smaller than an inside dimension of the container.

2. The device of claim 1 wherein the outer dimension of the disruption element is greater than 0.3 times said inner dimension of the container.

3. The device of claim 2 wherein the outer dimension of the disruption element is greater than 0.75 times said inner dimension of the container.

4. The device of any one of claims 1 to 3 wherein the disruption element comprises a dense material.

5. The device of claim 4, wherein the disruption element has a density above 7.0, preferably above 8.0.

6. The device of claim 4 or claim 5 wherein the disruption element comprises a steel or a material having equivalent or greater density.

7. The device of any one of claims 1 to 6 wherein the disruption element is a stainless steel ball having a diameter of about 6 mm and the container is a tube having an inner diameter of about 8 mm.

8. A method for disrupting cells or tissue comprising placing a sample comprising cells or tissue in a container, adding a nucleic acid stabilizing solution to said container, placing a disruption element into said container, and employing a disruption device for 45 seconds or less.

9. The method of claim 8 wherein the sample on which the disruption device is employed is a lymph node sample, from which supernatant is decanted and from which nucleic acid is extracted.

10. A method of extracting nucleic acids from a tissue or cell sample comprising placing a sample comprising cells or tissue in a container, adding a nucleic acid stabilizing solution to said container, placing a disruption element into said container, employing a disruption device for 45 seconds or less, removing the sample on which the disruption device is employed, and extracting nucleic acids therefrom.

11. The method of any one of claims 8 to 10 conducted intra-operatively.

12. The method of any one of claims 8 to 11 wherein the sample is lymph node tissue.

13. The method of any one of claims 8 to 12 which uses a device according to any one of claims 1 to 7.
